# EUROPEAN PATENT APPLICATION

(11) **EP 1 369 114 A1**
(43) Date of publication of application: **10.12.2003**
(21) Application number: 02012675.1
(22) Date of filing: 07.06.2002
(51) Int. Cl.: A61K 31/198, A61K 31/196, A61K 31/455, A61P 37/06

(54) **Use of tryptophan metabolites as pharmaceutical agents**

(71) Applicant: Terness, Peter, Priv. Doz. Dr., 69124 Heidelberg (DE); Bauer, Thomas, Dipl.-Ing., 69151 Neckargemünd (DE); Opelz, Gerhard, Prof. Dr., 69118 Heidelberg (DE)
(72) Inventor: Terness, Peter, Priv. Doz. Dr., 69124 Heidelberg (DE); Bauer, Thomas, Dipl.-Ing., 69151 Neckargemünd (DE); Opelz, Gerhard, Prof. Dr., 69118 Heidelberg (DE)
(74) Representative: Isenbruck, Günter, Dr.

(57) **Abstract**

The present invention relates to the use of tryptophan metabolites as pharmaceutical agents.

Kynurenine, N-formylkynurenine, anthranilic acid, quinolinic acid, 3-hydroxykynurenine, and / or 3-hydroxyanthranilic acid can be employed for the treatment of any disease, which requires immunosuppressive treatment such as the inhibition of T cell response.

Examples of such diseases are immunologically caused recurrent abortions, autoimmune or rheumatic diseases. Furthermore, they can be used for suppression of rejection in the case of cell, tissue, or organ transplantations.

## Description

The present invention relates to the use of tryptophan metabolites as pharmaceutical agents.

Indoleamine 2,3-dioxygenase (IDO) is the rate-limiting enzyme in the catabolism of tryptophan, expressed in a series of human and animal tissues, particularly in lymphoid organs and placenta ("Mellor et al.", 1999, Immunol. Today. 20:469-473). Apart from low level expression in healthy individuals, enzyme production markedly increases during infection or inflammation and can be induced by lipopolysaccharide (LPS), cytokines, or other agents ("Yoshida et al.", 1979, Proc. Natl. Acad. Sci. USA. 76:4084-4086; "Yoshida et al.", 1978, Proc. Natl. Acad. Sci. USA. 75:3998-4000; "Carlin et al.", 1987, J. Immunol. 139:2414-2418). Low tryptophan concentrations, which may be induced by IDO, are associated with inhibited proliferation of viruses, protozoan parasites, and other pathogens in eukaryotic cells and also with decreased proliferation of tumor cells ("Mellor et al.", 1999, Immunol. Today. 20:469-473). Based on these observations it has been postulated that the role of IDO in vivo is to inhibit the propagation of eukaryotic intracellular pathogens by depriving them of tryptophan.

Recent studies support the idea that, in addition to defense against pathogens, IDO participates in the regulation of T-cell responses ("Munn et al.", 1999, J. Exp. Med. 189:1363-1372). It was speculated that expression of IDO in antigen presenting cells of the immune system may control autoreactive T cells. Munn et al. demonstrated that IDO expression in the placenta is critically involved in preventing rejection of allogeneic fetuses ("Munn et al.", 1998, Science. 281:1191-1193). It is well known that, from an immunological viewpoint, pregnancy constitutes a paradoxical situation in which a foreign tissue is tolerated by the host. Munn et al. implanted time-release capsules containing the IDO-inhibitor 1-methyl-tryptophan under the skin of pregnant mice and observed that the fetus was rejected. Hints at a participation of T cells in fetal rejection came from the same study, in which immunodeficient pregnant mice were reconstituted with T cells specific for paternal major histocompatibility complex (MHC) antigens inherited by the fetus and thereafter treated with IDO inhibitor. Apparently, inhibition of IDO production in the placenta abrogated T-cell control and the pregnant mice lost their concepti.

This means that the measures which can be taken, for example, to avoid the rejection of fetuses (i.e. the treatment of recurrent abortion) or for the treatment of other diseases requiring T cell suppression, should be aimed at increasing the availability of IDO - an enzyme known to reduce tryptophan concentration. On the other hand, the role of the IDO-induced tryptophan metabolites in the suppression of T-, B-, and NK-cell activity has not been investigated so far.

WO 99/01125 relates to the use of a pharmaceutical composition for the treatment of malignant neoplasms and immunosuppressive deficiencies. Pharmaceutical compositions according to WO 99/01125 contain as active ingredients kynurenine associated with tymine or, alternatively, tryptophan associated with tymine. Interestingly, according to WO 99/01125 tryptophan and/or kynurenine seem to have a potential for activating the immune response, since the treatment of malignant neoplasms cannot be performed by administering medicaments which suppress the immune response.

The cytotoxic impact of two IDO-induced tryptophan metabolites (3-hydroxykynurenine, and. / or 3-hydroxyanthranilic acid) on lymphocytes was considered but could not be proved by Teulings et al. [Acta vitamin. enzymol. (Milano), 1975, 29, 113-116]. The use of said metabolites for the treatment of diseases requiring the suppression of T cell response is not anticipated by Teulings et al.

The object of the present invention is to provide an alternative to the common way of increasing the availability of IDO for treating diseases requiring immunosuppressive treatment.

This object is attained by administering at least one compound selected from the group consisting of kynurenine, N-formylkynurenine, anthranilic acid, quinolinic acid, 3-hydroxykynurenine, and 3-hydroxyanthranilic acid to a person in need of immunosuppressive treatment.

Furthermore, the present invention also comprises the administration of other IDO-induced tryptophan metabolites than kynurenine, N-formylkynurenine, anthranilic acid, quinolinic acid, 3-hydroxykynurenine, and 3-hydroxyanthranilic acid to a person in need of immunosuppressive treatment.

An advantage of the present invention is that the administration of kynurenine, N-formylkynurenine, anthranilic acid, quinolinic acid, 3-hydroxykynurenine, and/or 3-hydroxyanthranilic acid can be easily performed and it is therefore cheaper than increasing the availability of IDO.

Preferably, at least one compound selected from the group consisting of kynurenine, N-formylkynurenine, 3-hydroxykynurenine and 3-hydroxyanthranilic acid is administered to a person in need of immunosuppressive treatment.

Most preferably, at least one compound selected from the group consisting of kynurenine, 3-hydroxykynurenine and 3-hydroxyanthranilic acid is administered to a person in need of immunosuppressive treatment.

In a preferred embodiment of the present invention 3-hydroxykynurenine and 3-hydroxyanthranilic acid are administered in equal ratios.

The present invention also includes the use of all stereoisomeric forms of kynurenine, N-formylkynurenine or 3-hydroxykynurenine. Independently, they can have S configuration or it configuration and they can be present in enantiomerically pure form, both as levorotatory and as dextrorotatory antipodes, in the form of racemates and in the form of mixtures of the two enantiomers in all ratios. All these forms are an object of the present invention. The preparation of individual stereoisomers can be carried out, if desired, by separation of a mixture by customary methods, for example by chromatography or crystallization, by the use of stereochemically uniform starting materials for the synthesis or by stereoselective synthesis. Optionally a derivatization can be carried out before a separation of stereoisomers.

Kynurenine, N-formylkynurenine, anthranilic acid, quinolinic acid, 3-hydroxykynurenine, and 3-hydroxyanthranilic acid contain acidic or basic groups, the invention also comprises their corresponding pharmaceutically or toxicologically acceptable salts, in particular their pharmaceutically utilizable salts. Thus, acidic groups can be present according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Basic groups, i.e. groups which can be protonated, are according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. Since kynurenine, N-formylkynurenine, anthranilic acid, quinolinic acid, 3-hydroxykynurenine, and 3-hydroxyanthranilic acid simultaneously contain acidic and basic groups in the molecule, the invention also includes, in addition to the salt forms mentioned, inner salts or betaines (zwitterions). The respective salts can be obtained by customary methods which are known to the person skilled in the art like, for example by contacting these with an organic or inorganic acid or base in a solvent or dispersant, or by anion exchange or cation exchange with other salts. The present invention also includes all salts which, owing to low physiological compatibility, are not directly suitable for use in pharmaceuticals but which can be used, for example, as intermediates for chemical reactions or for the preparation of pharmaceutically acceptable salts.

The synthesis of kynurenine, N-formylkynurenine, anthranilic acid, quinolinic acid, 3-hydroxykynurenine, and 3-hydroxyanthranilic acid is well known to those skilled in the art.

Kynurenine, N-formylkynurenine, anthranilic acid, quinolinic acid, 3-hydroxykynurenine, and / or 3-hydroxyanthranilic acid can be employed for the treatment of any disease which is characterized by increased activity of the immune system and/or which requires immunosuppression such as the suppression of the response of T-, NK- and/or B-cells. Examples of such diseases are immunologically mediated recurrent abortions, autoimmune diseases, or rheumatic diseases. Furthermore, they can be employed for suppression of rejection in case of cell, tissue, or organ transplantations, preferred indications being kidney-, heart-, liver-, pancreas-, cornea-, and bone marrow transplantations.

Examples of autoimmune diseases include but are not limited to: Hashimoto's thyroiditis, primary myxoedema, Grave's disease, pernicious anaemia, autoimmune atrophic gastritis, Addison's disease, ovarian failure, premature menopause, insulin-dependent diabetes mellitus, Goodpasture's syndrome, myasthenia gravis, Lambert-Eaton syndrome, pemphigus, bulous pemphigoid, psoriasis, Crohn's disease, sympathetic ophthalmia, autoimmune uveitis, multiple sclerosis, autoimmune hemolytic anaemia, autoimmune thrombocytic purpura, autoimmune neutropenia, primary biliary cirrhosis, chronic active hepatitis, cryptogenic cirrhosis, ulcerative colitis, Sjögren's syndrome, antiphospholipid syndrome, mixed connective tissue disease, vasculitis, vitiligo, glomerulonephritis, etc.

Examples of rheumatic or rheumatoid diseases include but are not limited to: Diffuse diseases of connective tissue (e.g. systemic lupus erythematosus, nephritis, systemic sclerosis etc.), rheumatoid arthritis and inflammatory polyarthritis, osteoarthritis, spine disorders (e.g. ankylosing spondylitis), arthropathy, internal derangement of knee, joint disorders, symptoms involving skin and integumentary tissue, tendinitis and bursitis, disorders of synovium and of tendon and bursitis, disorders of muscle, ligament and fascia, disorders of soft tissue, crystal arthropathies, disorders of plasma protein metabolism, other inflammatory diseases (e.g. psoriatic arthritis, Lyme disease, Reiter's disease etc.).

Kynurenine, N-formylkynurenine, anthranilic acid, quinolinic acid, 3-hydroxykynurenine, and / or 3-hydroxyanthranilic acid can also be used in combination with other pharmaceutically active compounds, preferably compounds which suppress the immune response. Examples of such compounds include: Glucocorticoids (like cortisone, triamcinolon, prednisolone or prednisone), antimetabolites (like azathioprin or mycophenolate mofetil), calcineurin inhibitors (like cyclosporine A, tacrolimus, sirolimus, leflunomide), antibodies (monoclonal or polyclonal) against cells of the immune system (like anti-CD3, anti-thymocyte globulin, or anti-CD25), pooled human gamma globulin etc.

Kynurenine, N-formylkynurenine, anthranilic acid, quinolinic acid, 3-hydroxykynurenine, and / or 3-hydroxyanthranilic acid and their pharmaceutically acceptable salts, optionally in combination with other pharmaceutically active compounds, can be administered to animals, preferably to mammals, and in particular to humans, as pharmaceuticals by themselves, in mixtures with one another or in the form of pharmaceutical preparations.

Furthermore, subject of the present invention are pharmaceutical preparations (or pharmaceutical compositions) which comprise an effective dose of kynurenine and at least one compound selected from the group consisting of N-formylkynurenine, anthranilic acid, quinolinic acid, 3-hydroxykynurenine and 3-hydroxyanthranilic acid and a pharmaceutically acceptable carrier, i.e. one or more pharmaceutically acceptable carrier substances and/or additives. Within said pharmaceutical preparations kynurenine, N-formylkynurenine, anthranilic acid, quinolinic acid, 3-hydroxykynurenine, and / or 3-hydroxyanthranilic acid can also be present in form of their pharmaceutically acceptable salts.

Another subject of the present invention is the use of pharmaceutical preparations comprising an effective dose of at least one compound selected from the group consisting of kynurenine, N-formylkynurenine, anthranilic acid, quinolinic acid, 3-hydroxykynurenine, 3-hydroxyanthranilic acid and their pharmaceutically acceptable salts and a pharmaceutically acceptable carrier for the immunosuppressive treatment of recurrent abortions, autoimmune diseases, rheumatic diseases or other immunologically caused diseases as well as for the immunosuppressive treatment in case of cell, tissue and organ transplantations.

The pharmaceutical preparations according to the invention can be administered orally, for example in the form of pills, tablets, lacquered tablets, sugar-coated tablets, granules, hard and soft gelatin capsules, aqueous, alcoholic or oily solutions, syrups, emulsions or suspensions, or rectally, for example in the form of suppositories. Administration can also be carried out parenterally, for example subcutaneously, intramuscularly or intravenously in the form of solutions for injection or infusion. Other suitable administration forms are, for example, percutaneous or topical administration, for example in the form of ointments, tinctures, sprays or transdermal therapeutic systems, or the inhalative administration in the form of nasal sprays or aerosol mixtures, or, for example, microcapsules, implants or rods. The preferred administration form depends, for example, on the disease to be treated and on its severity.

The amount of kynurenine, N-formylkynurenine, anthranilic acid, quinolinic acid, 3-hydroxykynurenine, and / or 3-hydroxyanthranilic acid and/or its pharmaceutically acceptable salts in the pharmaceutical preparations normally ranges from 2mg to 10g, preferably from 5mg to 5g, in particular from 1g to 2g per dose, but depending on the type of the pharmaceutical preparation it may also be higher. The pharmaceutical preparations usually comprise 0.5 to 90 percent by weight of kynurenine, kynurenine, N-formylkynurenine, anthranilic acid, quinolinic acid, 3-hydroxykynurenine, and / or 3-hydroxyanthranilic acid and/or their pharmaceutically acceptable salts. The preparation of the pharmaceutical preparations can be carried out in a manner known per se. To this end, kynurenine, N-formylkynurenine, anthranilic acid, quinolinic acid, 3-hydroxykynurenine, and / or 3-hydroxyanthranilic acid and/or their pharmaceutically acceptable salts, together with one or more solid or liquid pharmaceutical carrier substances and/or additives (or auxiliary substances) and, if desired, in combination with other pharmaceutically active compounds having therapeutic or prophylactic action, are brought into a suitable administration form or dosage form which can then be used as a pharmaceutical in human or veterinary medicine.

For the production of pills, tablets, sugar-coated tablets and hard gelatin capsules it is possible to use, for example, lactose, starch, for example maize starch, or starch derivatives, talc, stearic acid or its salts, etc. Carriers for soft gelatin capsules and suppositories are, for example, fats, waxes, semisolid and liquid polyols, natural or hardened oils, etc. Suitable carriers for the preparation of solutions, for example of solutions for injection, or of emulsions or syrups are, for example, water; physiologically sodium chloride solution, alcohols such as ethanol, glycerol, polyols, sucrose, invert sugar, glucose, mannitol, vegetable oils, etc. It is also possible to lyophilize kynurenine, N-formylkynurenine, anthranilic acid, quinolinic acid, 3-hydroxykynurenine, and / or 3-hydroxyanthranilic acid and their pharmaceutically acceptable salts and to use the resulting lyophilisates, for example, for preparing preparations for injection or infusion. Suitable carriers for microcapsules, implants or rods are, for example, copolymers of glycolic acid and lactic acid.

Besides kynurenine, N-formylkynurenine, anthranilic acid, quinolinic acid, 3-hydroxykynurenine, and / or 3-hydroxyanthranilic acid and carriers, the pharmaceutical preparations can also contain additives, for example fillers, disintegrants, binders, lubricants, wetting agents, stabilizers, emulsifiers, dispersants, preservatives, sweeteners, colorants, flavorings, aromatizers, thickeners, diluents, buffer substances, solvents, solubilizers, agents for achieving a depot effect, salts for altering the osmotic pressure, coating agents or antioxidants.

The dosage of kynurenine, N-formylkynurenine, anthranilic acid, quinolinic acid, 3-hydroxykynurenine, and / or 3-hydroxyanthranilic acid to be administered and/or of a pharmaceutically acceptable salt thereof depends on the individual case and is, as is customary, to be adapted to the individual circumstances to achieve an optimum effect. Thus, it depends on the nature and the severity of the disorder to be treated, and also on the sex, age, weight and individual responsiveness of the human or animal to be treated, on the efficacy and duration of action of the compounds used, on whether the therapy is acute or chronic or prophylactic, or on whether other active compounds are administered in addition to kynurenine, 3-hydroxykynurenine, and / or 3-hydroxyanthranilic acid. In general, a daily dose of approximately 0.01 to 200 mg/kg, preferably 0.1 to 20 mg/kg, in particular 0.3 to 10 mg/kg (in each case mg per kg of bodyweight) is appropriate for administration to an adult weighing approximately 75 kg in order to obtain the desired results. The daily dose can be administered in a single dose or, in particular when larger amounts are administered, be divided into several, for example two, three or four individual doses. In some cases, depending on the individual response, it may be necessary to deviate upwards or downwards from the given daily dose.

The present invention will now be illustrated in the following examples:

### Examples:

### 1) In vitro experiments

### General Methods

### Construction of replication-defective recombinant IDO-adenoviruses.

Replication-defective adenoviruses were generated by encoding cDNA for human IDO according to a method previously described ("He et al.", 1998, Proc. Natl. Acad. Sci. USA, 95:2509-2514). The human IDO gene was inserted into a shuttle plasmid (pAdTrack-CMV, harboring the green fluorescence protein gene, or pShuttle-CMV, without the GFP gene)(Q-Biogene, Carlsbad, CA). The resultant plasmid was linearized by digestion with restriction endonuclease PmeI and subsequently cotransformed with an adeno-5 virus backbone (pAdEasy-1) into E.coli cells BJ5183. Recombinants were selected for kanamycin resistance, and recombination confirmed by restriction digest with Pad. The relevant clones yielded a large fragment (near 30kb), plus a smaller fragment (3.0kb or 4.5kb). Finally, the linearized recombinant IDO plasmid was transfected by Effectene Transfection Reagent Kit (Qiagen GmbH, Hilden, Germany) into adenovirus packaging cells (911 cells = E1-transformed human embryonic retinoblast cell line) at 50% confluency. In case of pAdTrack-based vectors, transfection and viral production can be monitored by GFP expression. When a cytopathogenic effect becomes evident (usually 7-10 days post-transfection), the cells are scraped off, repeatedly dounced, and centrifuged. Part of the supernatant is used for further multiplication of viruses. The viruses were purified via ultracentrifugation on CsC1-gradient, rebuffered in conservation buffer, titrated, and kept at -80°C until use. Titration was performed by limiting dilution of viral suspension on 911 cells. The last "infective concentration" was determined by checking the GFP expression - in case of pAdTrack-CMV constructs, or by PCR with adenoviral-specific primers - in case of pShuttle-CMV constructs. Two recombinant vectors were generated, IDO-adeno-GFP (with the GFP gene) and IDO-adeno (without GFP). The nucleotide IDO sequence of recombinant adenoviruses was determined using the dideoxy termination method of Sanger. Sequence analysis was performed in an ABI-3077 sequencer (Applied Biosystems Inc.) and identity with the sequence of the gene bank was ascertained. Transcription of the IDO gene in transfected eukaryotic cells was verified by RT-PCR. Briefly, mRNA was extracted from IDO-adeno-infected 911 cells (QuickPrep Micro mRNA Purification Kit, Amersham Biosciences Europe, Freiburg, Germany), transcribed with Moloney virus leukemia reverse transcriptase into cDNA (Ready-to-go-Primed First-Strand Kit, Amersham Biosciences Europe), and amplified by PCR with IDO-specific primers. PCR products were analyzed by agarose gel electrophoresis with ethidium bromide staining.

### Generation of human dendritic cells (DCs).

DCs were generated from CD14-positive blood monocytes as previously described ("Schuler G. and N. Romani", 1997, Adv. Exp. Med. Biol. 417:7-13). Briefly, peripheral blood mononuclear cells of an HLA-typed healthy donor were separated by density gradient centrifugation and CD14-positive monocytes were isolated by adherence to culture dishes. Non-adherent cells were discarded. After culture of monocytes in the presence of human rGM-CSF (666 Units/ml) and rIL-4 (1000 Units/ml) for 7 days, rTNF-α (10 ng/ml) was added for an additional 2 days to induce maturation of DC. Characterization of DC phenotype was performed by flow cytometry using the following mouse monoclonal antibodies: anti-CD 14-FITC, anti-HLA-DR-FITC, anti-CD 1a-PE, anti-CD86-PE, and anti-CD83-PE (BD Biosciences Heidelberg, Germany). DCs were kept in liquid nitrogen until use.

### Separation oflymphocyte subpopulations.

Cells were separated by negative selection via magnetic beads. Lymphocytes (PBMCs) were obtained from blood of healthy donors by density gradient centrifugation, washed, and incubated at +4°C for 10 min. with monoclonal mouse antibody mixes directed against lymphocyte subpopulations (10⁷ cells + 20µM antibody mix). Thereafter, the cells were washed and incubated at +4°C, for 10 min. with 50µl magnetic beads coated with Fc-specific antibodies (Dynabeads, Dynal A.S., Oslo, Norway). Antibody-labeled cells were removed with a magnet. In order to increase the purity of the remaining lymphocyte subpopulation, the procedure was repeated with another portion of magnetic beads. The purity of negatively selected cells was for T cells 85-90%, NK cells 70-80%, and B cells 90%.

### Cell cultures.

(a) Allogeneic T-cell response. DCs were infected with recombinant IDO-adeno-GFP (DC-IDO-GFP), IDO-adeno (DC-IDO) or with control adenoviruses (DC-adeno) at a MOI (multiplicity of infection) up to 500 for 2 days. Lymphocytes (PBMCs) were separated from peripheral blood of healthy donors by density gradient centrifugation and typed for HLA antigens. Only HLA-DR-different responder lymphocytes were used for DC-induced stimulation. Native DCs only, lymphocytes only, native DCs + allogeneic lymphocytes, DC-IDO-GFP + allogeneic lymphoytes, DC-IDO + allogeneic lymphoytes, DC-adeno + allogeneic lymphocytes (each time 10⁵ DC + 10⁵ lymphocytes in 0.2ml medium) were cultured in RPMI 1640 medium (Gibco BRL Life Technologies GmbH, Eggenstein, Germany) with 10% fetal calf serum (FCS) (final tryptophan concentration 5.25 mg/l = 25.69 uM) and Penicillin G/Streptomycin. After 5 days, ³[H]-thymidin was added for 12hrs and the number of counts (counts per minute = cpm) determined in a (3-counter (Inotech Biosystems, Lansing, MI).
   In order to verify whether IDO expression or adenovirus infection harm the DCs, 10⁵ cells were infected with IDO-adeno-GFP, IDO-adenovirus, or adenovirus only at a stepwise decreasing MOI (500, 250, 125, ........ 3.9) and cell viability was measured in FACS following double staining with 7-AAD and anti-CD83 antibody-PE. The percentage of dead cells never exceeded 30% for any MOI following IDO-adeno-GFP- or IDO-adeno-infection and 40% following adenovirus(control)-infection. Moreover, these values were not higher than those of native DCs cultured for the same period of time. The experiment was repeated using ethidium bromide or trypan blue staining with virtually the same result. These findings show that neither IDO-expression nor adenovirus infection harm DCs under the described conditions.
(b) CD3 stimulation of T cells. Peripheral blood lymphocytes were cultured (10⁵ cells/well) in RPMI 1640 (10% FCS) with anti CD3 monoclonal antibody (Ortho Diagnostic Systems Inc., Raritan, NJ) (final dilution 1:200). After 2 days, cell proliferation was assessed by measuring ³[H]-thymidin incorporation (cpm) as described.
(c) Tryptophan metabolite titration curves. Kynurenine, 3-hydroxykynurenine, anthranilic acid, 3-hydroxyanthranilic acid, and quinolinic acid (Sigma-Aldrich Chemie GmbH, Taufkirchen, Germany)(for all substances purity>98%) were dissolved in RPMI 1640 and added to cell cultures at various concentrations. In an other experiment, to determine whether the metabolites have an additive effect, equal concentrations of the compounds were mixed and added to the cultures. After 2 days, ³[H] thymidin was added and the number of counts recorded. Positive controls consisted of CD3-stimulated cells without metabolites and negative controls of unstimulated cells.
(d) Restimulation of suppressed T cells. Lymphocytes (10⁵ cells/well) were cultured in RPMI 1640 (10% FCS) with anti-CD3 monoclonal antibody (final dilution 1:200) and kynurenine (dilution series). After 2 days the cells were washed, the cell culture medium (RPMI 1640 + 10%FCS) was replaced, and the cells were stimulated with Concanavalin A (6.25µg/ well). After another 2 days, ³[H] thymidin was added and cpm determined. Controls consisted of cells prestimulated with anti-CD3 but not pretreated with kynurenine. In an other restimulation experiment lymphocytes were stimulated with allogeneic DCs as described, in the presence (47-1500µM) or absence of kynurenine. Control cultures contained DCs or lymphocytes only. After 5 days the cells were extensively washed, and restimulated with an identical number of DCs derived from the same donor or from third party donors. 3[H] thymidin was added to all cultures and the number of counts (cpm) determined.
(e) Tryptophan titration curve. In order to define the concentration of tryptophan which reduces T- lymphocyte proliferation in our test system, 10⁵ lymphocytes were incubated in tryptophan-free RPMI 1640 medium (Gibco BRL Life Technologies) with or without 10% FCS, stimulated with anti-CD3 monoclonal antibody, and various amounts (0-38µM) of tryptophan were added. Unimpaired proliferation was observed down to 0.30µM (medium with FCS) and 1 .20µM (without FCS). Complete inhibition was not obtained at any concentration (positive control 100% proliferation; maximal suppression in medium with FCS: approx. 66% and without FCS: approx. 33%). All cell cultures were done in triplicate.

### Determination of tryptophan and kynurenine concentration in cell cultures.

Native DC (control), DC-IDO-GFP, DC-IDO, or DC-adeno (control) were cultured under conditions described above and tryptophan and kynurenine were measured at the end of culture. Supernatant (20µl) was collected and the tryptophan concentration was defined using reversed-phase HPLC (RP18 Hypersil ODS column) (Supelco Inc., Bellefonte, PA) and photometric detection (fluorescence at 278/363 nm). In another test the cells were lysed, centrifuged, and the kynurenine concentration was measured in the supernatant spectrophotometrically. Briefly, 75µl supernatant was incubated with 125µl methylene blue reaction buffer for 1hr at 37C, and the reaction was stopped with 30% trichloroacetic acid. After centrifugation, 125µl supernatant was incubated with 125µl Ehrlich reagent for 10 min at room temperature in a microtiter plate. Optical density was measured at 480nm.
The values were referred to a standard curve with defined kynurenine (0-120 µM) concentrations.

### Detection of cell death.

Lymphocytes were stimulated in culture with anti-CD3 monoclonal antibody (s. above) in the presence or absence (control) of kynurenine (1501µM), 3-hydroxyanthranilic acid (510µM), 3-hydroxykynurenine (348µM), or metabolite mixture (64µM kynurenine + 64µM 3-hydroxykynurenine + 64µM anthranilic acid + 64µM 3-hydroxyanthranilic acid + 64pM quinolinic acid). After 3 days the cells were washed and resuspended in PBS (10⁶/ml). Thereafter, the cells (100µ1 suspension) were labeled with anti-CD3-FITC antibody (3 µl), stained with 7-AAD (5µl) for 15 min, and the percentage of 7-AAD-positive (dead) T cells was determined in a FACScan. In an other experiment T cells, B cells, NK-cells, or DCs were separated from PBMCs by magnetic beads or generated as described, and cultured (5x10⁵ cells/ml) with a mixture of kynurenine metabolites (kynurenine + 3-hydroxykynurenine + anthranilic acid + 3-hydroxyanthranilic acid + quinolinic acid)(8, 16, or 32µM respectively for each compound). In one setting T cells were activated with anti-CD3 antibody. Cell death was measured daily for 5 days by 7-AAD staining and expressed in percentage of dead cells.

### Statistics.

All tests were carried out in triplicate and results are given as mean +standard deviation. For calculating half-inhibitory (I₅₀) concentrations of substances, the mean proliferation (cpm) of stimulated and completely suppressed cells was determined. Thereafter, the concentration of substance giving 50% proliferation was calculated by regression.

### Results

### IDO-gene expression in human dendritic cells.

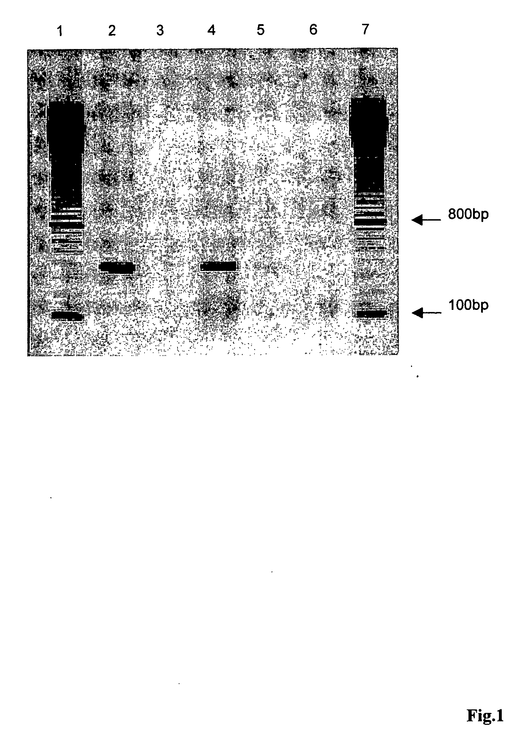

Human IDO cDNA was inserted into a shuttle vector and recombined with an adeno-5 backbone vector in bacteria as previously described ("He et al.", 1998, Proc. Natl. Acad. Sci. USA, 95:2509-2514). The replication-defective recombinant IDO adenoviruses were multiplied in 911 cells. Sequence analyses confirmed the correct sequence of human IDO (data not shown) and RT-PCR with IDO-specific primers proved the transcription of IDO transgene in eukaryotic cells infected with recombinant adenoviruses. Fig. 1 shows the transcription of IDO-gene in eukaryotic cells infected with recombinant IDO-adenoviruses. mRNA was extracted from human embryonic retinoblast cells infected with IDO-adeno-GFP, IDO-adeno, or with replication-defective adenoviruses (neg. ctr.-a) and reverse transcribed into cDNA. PCR with IDO-specific primers was performed and the products analyzed by agarose gel electrophoresis. Positive control consisted of material extracted from recombinant IDO-adenoviruses and neg. ctr.-b of water instead of template. Lane: 1, DNA molecular weight marker; 2, pos. ctr.; 3, neg. ctr.-b; 4, IDO-adeno-GFP; 5, IDO-adeno; 6, neg. ctr.-a; 7, DNA molecular weight marker. Lane 4 and 5 show the relevant IDO-specific bands.

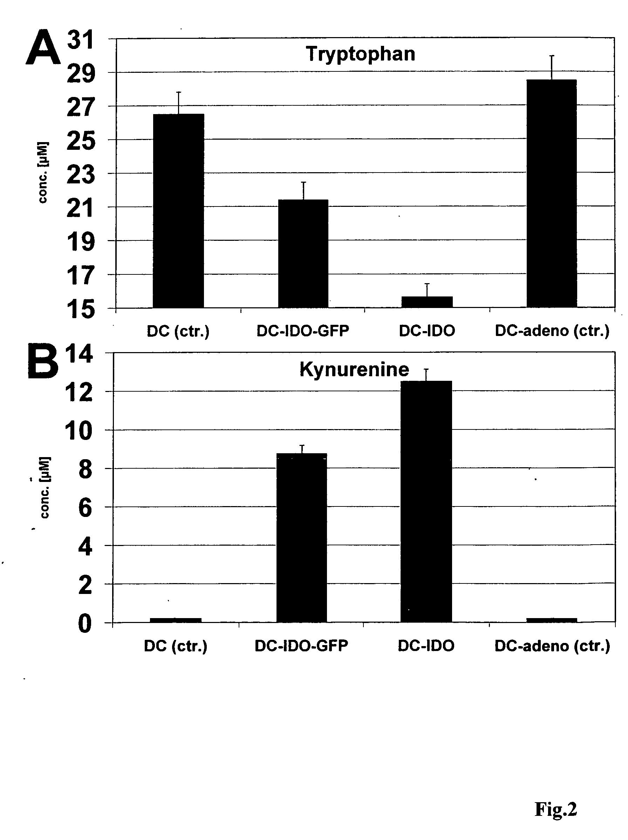

DCs were generated from blood monocytes in the presence of GM-CSF and IL-4, maturation was induced by TNF-a, and the phenotype was verified by FACS analysis ("S'chuler, G. and N. Romani ", 1997, Adv. Exp. Med. Biol., 417:7-13). These cells were infected with the recombinant IDO-adenoviruses. The efficiency of infection was monitored by expression of the green fluorescence marker protein harbored by the virus. Vital staining using FACS analysis showed that DCs remained viable following adenoviral infection. It is known that IDO catalyzes the oxidation of tryptophan, resulting in kynurenine and other metabolites. In order to verify whether transgenic IDO exerted its catabolic effect, tryptophan as well as kynurenine were measured in cultures of DCs infected with IDO-adenoviruses. As expected, the tryptophan concentration significantly decreased while kynurenine increased. Fig 2 shows the tryptophan and kynurenine concentration in cultures of IDO-expressing dendritic cells. Native DCs (control), DCs infected with IDO-adeno-GFP, IDO-adeno, or native replication-defective adenoviruses (control) cultured in RPMI 1640 plus 10% FCS. Tryptophan (a) and kynurenine (b) concentrations were determined and the values (uM/10⁵ cells) are shown on the ordinate.

### IDO-expressing DCs reduce allogeneic T-cell response.

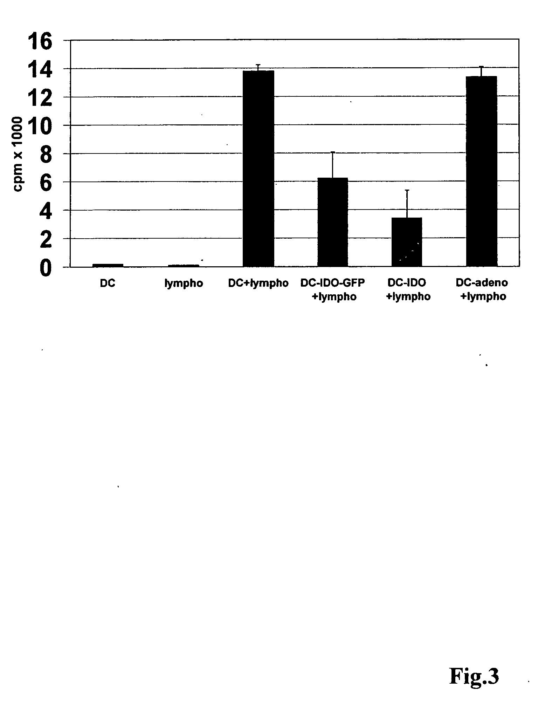

The next experiment addressed the question whether IDO-expressing DCs inhibit the response of HLA-incompatible T cells. Native DCs or IDO-expressing DCs were coincubated with allogeneic T cells and proliferation was determined by ³[H] thymidin incorporation. A significant reduction of response was noted by coincubating T cells with IDO-DCs, demonstrating the inhibitory effect of IDO on allogeneic T cells. Fig. 3 shows the reduction of allogeneic T-cell stimulation by IDO-expressing dendritic cells. Native DCs (pos. ctr.), DCs infected with IDO-adeno-GFP, IDO-adeno, or replication-defective adenoviruses (control) were coincubated with allogeneic peripheral blood lymphocytes for 6 days and cell proliferation measured by ³[H]-thymidin incorporation (cpm) (ordinate). Negative controls consisted of DCs or lymphocytes only.

### Kynurenine inhibits T-cell response.

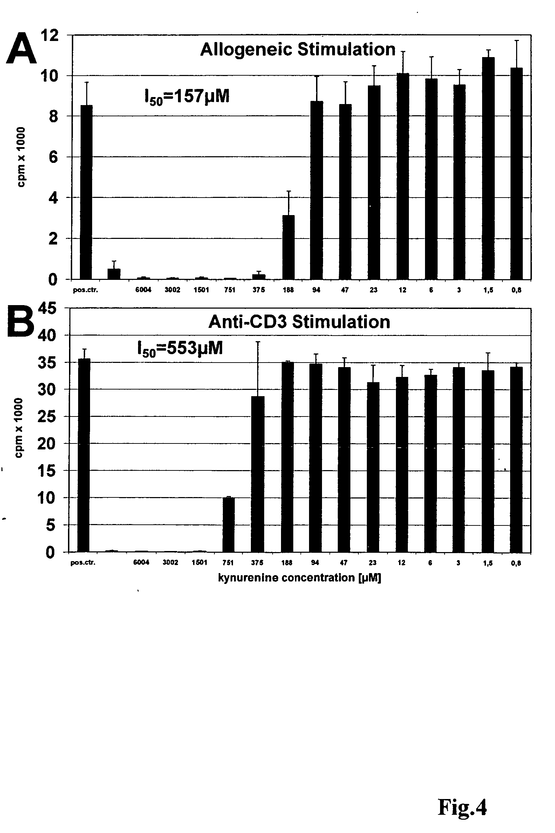

Previous studies came to the conclusion that tryptophan depletion is the mechanism by which IDO inhibits T cell activation ("Munn et al.", 1999, J. Exp. Med. 189:1363-1372). An alternative mechanism would be that metabolites resulting from tryptophan breakdown influence T cell responsiveness. IDO-induced tryptophan degradation results in N-formylkynurenine, kynurenine, 3-hydroxykynurenine, anthranilic acid, 3-hydroxyanthranilic acid, quinolinic acid, and other metabolites, whereby kynurenine is the main product. Therefore, it was first tested the influence of kynurenine on the allogeneic T-cell response. Fig. 4 shows the effect of kynurenine on T cell response induced by allogeneic dendritic cells or anti-CD3 antibody. Peripheral lymphocytes were stimulated with (A) allogeneic DCs or (B) anti-CD3 monoclonal antibody for 6 and 3 days, respectively. Various amounts of kynurenine (abscissa) were added to the cultures. Positive control was carried out without kynurenine. T-cell proliferation was determined by ³[H]-thymidin incorporation (cpm) (ordinate). As shown in Fig. 4A, L-kynurenine was able to completely inhibit the proliferation of T cells stimulated by allogeneic DCs (50% inhibition=150: 157µM). Moreover, L-kynurenine also suppressed proliferation of CD3-stimulated T cells (I₅₀: 553µM) (Fig. 4B). The same effect was noted with D-kynurenine (data not shown).

### Kynurenine-suppressed T cells cannot be restimulated.

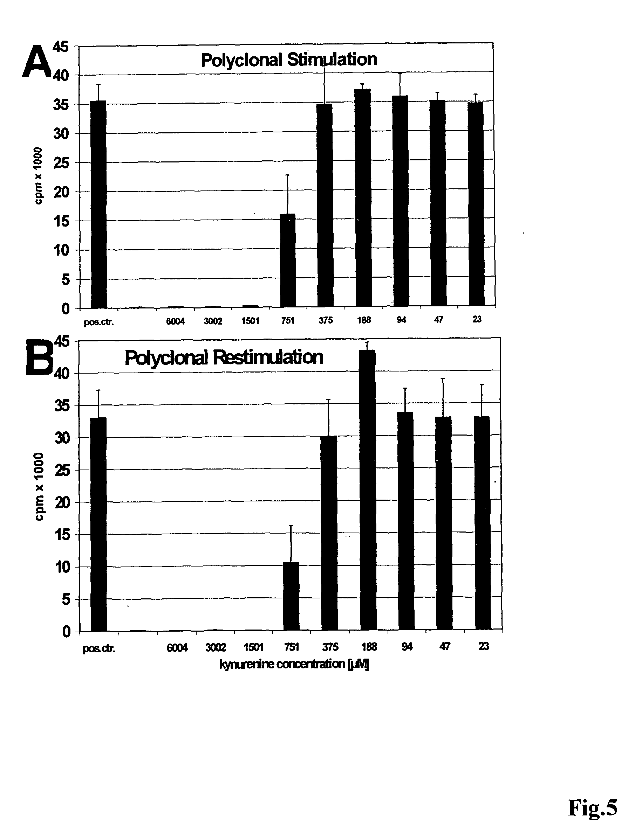

In the experiment depicted in Fig. 5A,B, T cells were stimulated with anti-CD3 antibody in the presence of various amounts of kynurenine, extensively washed, and restimulated with Con-A in the absence of kynurenine. Fig. 5 shows the polyclonal restimulation of kynurenine-suppressed T cells. Peripheral lymphocytes were stimulated with (A) anti-CD3 monoclonal antibody in the presence of various amounts of kynurenine (abscissa). Positive control consisted of T-cell stimulation in the absence of kynurenine. After 2 days the cells were washed and (B) restimulated with Con-A for an other 3 days. T-cell proliferation was determined by ³[H]-thymidin incorporation (cpm) (ordinate). The results show that kynurenine-suppressed T cells cannot be restimulated.

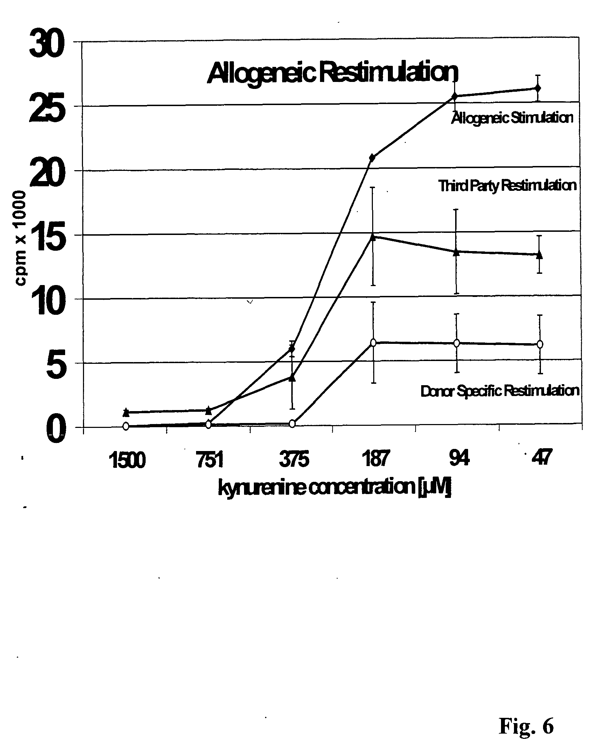

In a second experiment the question is addressed whether T cells stimulated with allogeneic DCs in the presence of kynurenine can be restimulated with donor-specific or third party DCs. Fig. 6 shows allogeneic restimulation of T cells activated with allogeneic dendritic cells. Peripheral blood lymphocytes were stimulated with allogeneic DCs in the presence of decreasing amounts of kynurenine (abscissa). After 5 days the cells were extensively washed and restimulated with DCs from the same or unrelated donors. T-cell proliferation was measured by 3[H]-thymidin incorporation (cpm) (ordinate) after primary an secondary stimulation. The curves show the degree of proliferation after the first stimulation (upper curve) and upon restimulation with third-party (intermediate) or donor-specific DCs (lower curve).

As expected, T cells directed against a certain donor, once suppressed cannot be restimulated with DCs from the same donor (s. Fig. 6, upper and lower curve at 1500, 751, and 751µM). Third-party DCs, however, are able to restimulate the T cells (s. medium curve). The degree of restimulation by third-party donors depends on the dose of kynurenine to which the cells were exposed. At extremely high doses (1500µM or 750µM) only minimal reactions was noted, whereas at medium or low doses (375µM - 47µM) significant restimulation takes place. Most importantly, at any kynurenine concentration, third-party restimulation (medium curve) was significantly higher than donor-specific restimulation (lower curve). This finding suggests that kynurenine preferentially compromises activated T cells.

### Other tryptophan metabolites also suppress the T-cell response.

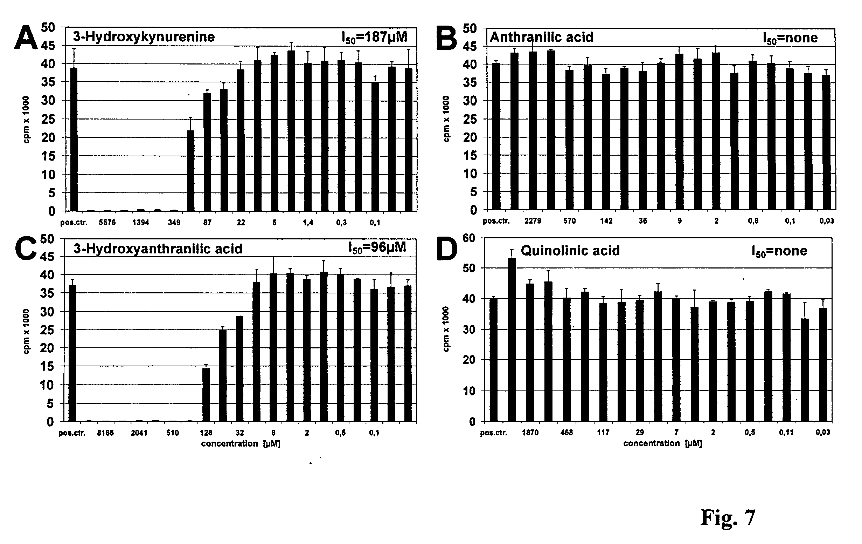

The question arose whether, in addition to kynurenine, other tryptophan metabolites also inhibit T cell response. Fig. 7 shows the effect of main IDO-induced tryptophan metabolites on T cell response. Peripheral lymphocytes were stimulated with anti-CD3 antibody for 3 days in the presence of various amounts (abscissa) of (A) 3-hydroxykynurenine, (B) anthranilic acid, (C) 3-hydroxyanthranilic acid, or (D) quinolinic acid. Positive control consisted of T-cell stimulation in the absence of metabolites. T-cell proliferation was determined by 3[H]-thymidin incorporation (cpm) (ordinate).

As shown in Fig. 7A-D, 3-hydroxykynurenine and 3-hydroxyanthranilic acid both strongly suppress T cells, whereas anthranilic- and quinolinic acid as well as other tryptophan metabolites (data not shown) were also suppressive but at higher concentrations. In order of their effectiveness, the T cell active components of tryptophan catabolism were: 3-hydroxyanthranilic acid (I₅₀: 96µM), 3-hydroxykynurenine (I₅₀=187µM), and kynurenine (150=553 µM).

### Tryptophan metabolites have an additive effect on T cells.

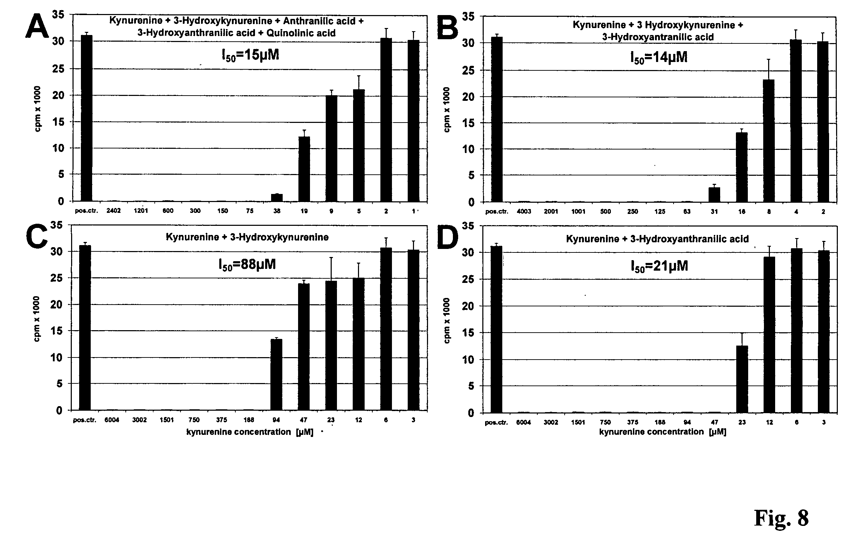

In vivo as well as in cell cultures, none of the tryptophan metabolites acts as a single component but in the presence of other metabolites. Fig. 8 shows the additive effect of tryptophan metabolites. Peripheral lymphocytes were stimulated with anti-CD3 antibody for 3 days and decreasing amounts of (A) kynurenine+3-hydroxykynurenine+anthranilic acid+3-hydroxyanthranilic acid+quinolinic acid, (B) kynurenine+3-hydroxykynurenine+3-hydroxyanthranilic acid, (C) kynurenine+3-hydroxykynurenine, (D) kynurenine+3-hydroxyanthranilic acid were added. All components of a mixture were added in equal amounts. The concentration of single substances is shown (abscissa).

Fig. 8A shows the action of kynurenine in combination with all other metabolites. It is evident that much smaller concentrations of kynurenine are required when combined with equal amounts of the other compounds (in combination: I₅₀=15µM, as single substance: I₅₀=553µM, s. Fig. 4B). Even the combination of 3 or 2 active components enhances the effect of single substances (Fig. 8 B-D). For instance, whereas kynurenine is effective at 553µM (s. Fig. 5B) and 3-hydroxyanthranilic acid at 96µM (s. Fig. 7A) as single components, when combined (s. Fig. 8D), 21 µM of each substance is sufficient to induce the same effect. Other combinations showed similar results, indicating that the metabolites add or even potentiate their respective effects.

### Kynurenine, 3-hydroxykynurenine, 3-hydroxyanthranilic acid, and the metabolite mixture induce T-cell death.

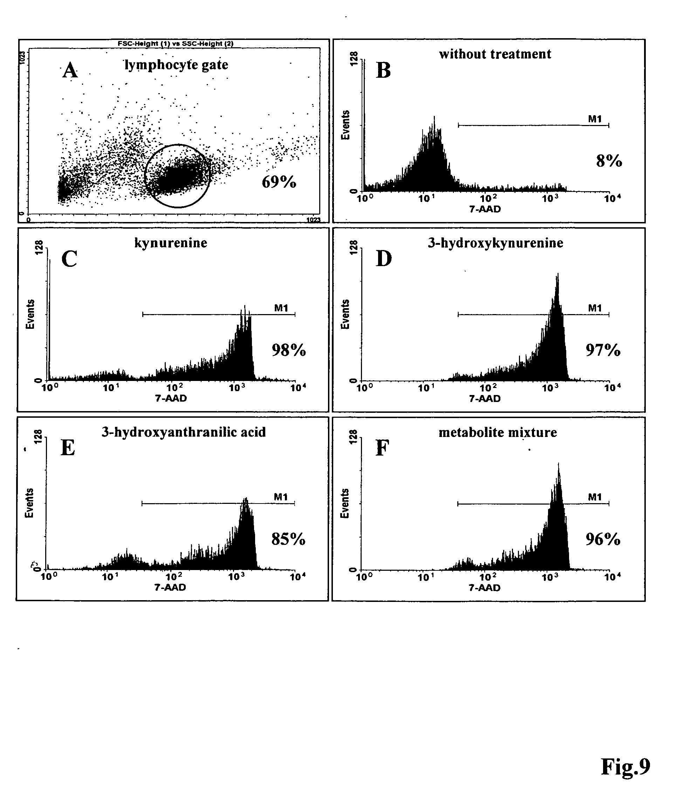

The lack of restimulation of kynurenine-suppressed T cells might be attributable to either T-cell areactivity or cell death. To analyze the underlying mechanism, the cells were stimulated as described in the presence of suppressive amounts of kynurenine, 3-hydroxykynurenine, 3-hydroxyanthranilic acid, or a mixture of main metabolites for 3 days, and cell viability was determined in FACS by vital staining with 7-AAD. Fig. 9 shows the induction of cell death by tryptophan metabolites. Lymphocytes were stimulated with anti-CD3 in the presence or absence (control) of active metabolites (1501 µM kynurenine, 349µM 3-hydroxykynurenine, 510µM 3-hydroxyanthranilic acid) or a metabolite mixture (kynurenine+3-hydroxykynurenine+anthranilic acid+3-hydroxyanthranilic acid+quinolinic acid)(64 µM of each compound). After 3 days the cells were washed, stained with anti-CD3-FITC antibody and 7-AAD. The percentages of dead (7-AAD-positive) T cells was determined in FACScan. Panel A shows the lymphocyte gate. Panel B-F show the percentage of dead cells in the negative control and after treatment with kynurenine, 3-hydroxyanthranilic acid, 3-hydroxykynurenine, or metabolite mixture. As shown in Fig. 9, virtually all cells died at suppressive concentrations of single components or the metabolite mixture.

### Tryptophan metabolites have a time-dependent cytotoxic effect.

In contrast to cell cultures, the exposure of lymphocytes to kynurenine derivatives "in vivo" is long-lasting. If the cytotoxic effect increases with time, even small amounts of metabolites may be effective. To test whether cytotoxicity is time-dependent, purified T cells were incubated for 5 days with various amounts of tryptophan metabolites and the percentage of dead cells was determined daily.

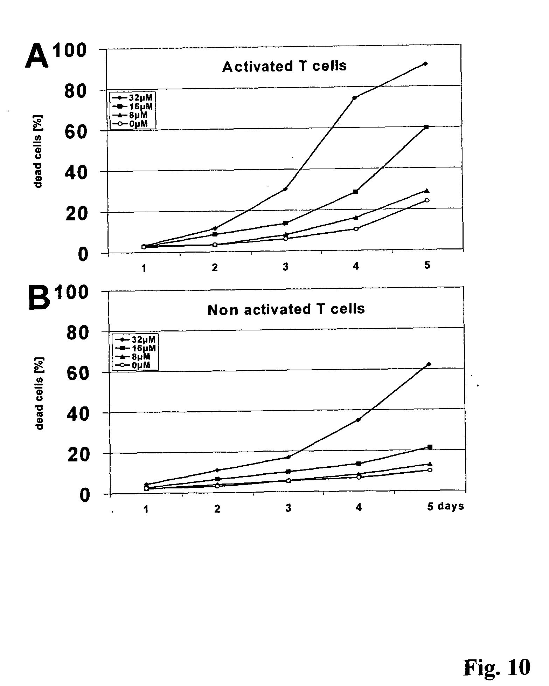

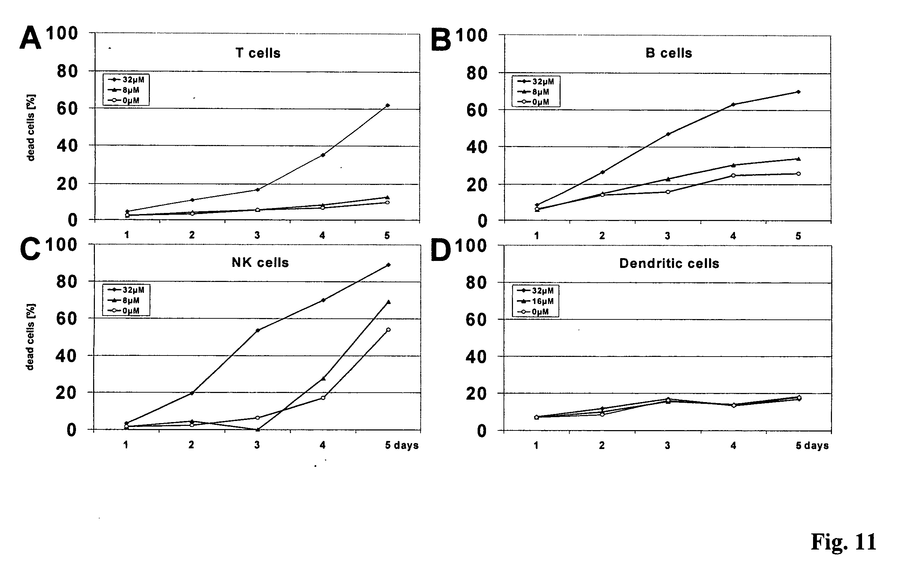

Fig. 10 shows the time-dependency of cytotoxicity. T cells were separated from PBMCs using magnetic beads and kept in culture (A) with anti-CD3 antibody or (B) without anti-CD3 antibody, in the presence of a mixture of tryptophan metabolites (kynurenine+3-hydroxykynurenine+anthranilic acid+3-hydroxyanthranilic acid+quinolinic acid)(concentrations: 0,8,16,32 uM for each compound). The percentage of dead cells (ordinate) was measured every day (abscissa) by 7-AAD staining. The findings clearly show that the cytotoxic effect is time-dependent and that even small amounts of substances become cytotoxic after an exposure time of several days. As shown in Fig. 11B,C, this is also the case for B- and NK cells.

### Tryptophan metabolites preferentially kill activated T cells

An important question is whether activated T cells are more susceptible to cell death than resting ones. Fig. 10A,B shows the metabolites' action on activated versus non-activated T cells. Evidently, activated cells are more sensitive. For instance, whereas 16pM metabolite mixture killed 60% activated T cells, it affected only 21% non-activated cells after 5 days of culture.

### Tryptophan metabolites act on several lymphocyte subpopulations

The effect of tryptophan metabolites on lymphocyte subpopulations was extensively analyzed. Fig. 11 shows the sensitivity of T cells, B cells, NK cells and dendritic cells to tryptophan metabolites. T-, B-, and NK cells were separated from PBMCs and DCs were generated as described. The cells were incubated with a metabolite mixture (kynurenine+3-hydroxykynurenine+anthranilic acid+3-hydroxyanthranilic acid+quinolinic acid)(concentrations: 0, 8, 16, 32µM for each metabolite). The percentage of dead cells (ordinate) was measured by 7-AAD-staining every day (abscissa).

T-, B-, and NK cells were separated by magnetic beads from PBMCs and incubated for 5 days with various amounts of metabolite mixture. The results show that all 3 subpopulations are sensitive to these compounds. Interestingly, DCs - the cells which produce the IDO and thus generate kynurenine - are resistant to cell death. This is in line with our observations showing that IDO-adenovirus-transfected DCs are not harmed more than mock-transfected or untreated cells

The above described experiments clearly demonstrate that IDO-expressing DCs decrease the concentration of tryptophan, increase the concentration of kynurenine, the main tryptophan metabolite, and suppress T cell response in vitro. Suppression of T-cell response can also be achieved by directly adding N-formylkynurenine, kynurenine, anthranilic acid, 3-hydroxykynurenine, 3-hydroxyanthranilic acid, and/or quinolinic acid to the cells, the suppressive effect being additive. T cells, once stopped in their proliferation, can not be restimulated. Inhibition of proliferation is at least partly due to T-cell death because at certain concentrations tryptophan metabolites exert a cytotoxic action on CD3⁺ cells. This action preferentially affects activated T cells and increases gradually with exposure time. In addition to T cells, B- and NK-cells are also killed, whereas DCs are not affected.

### 2) In vivo experiments

### Prevention of recurrent abortion by treatment with tryptophan metabolites

Murine CBA/JxDBA/2 matings are known to have a high spontaneous abortion rate which is enhanced by endotoxin treatment or stress. The abortion is mediated by T cells ("Arck P. et al.", 1999, Cell. Immunol. 196:71-79). CBA/J females are mated with DBA/2 male mice. One group is exposed to ultrasonic stress for 24 hrs. beginning on day 5 to boost abortion rates. Pregnant mice are treated daily with metabolites (intraperitoneally=i.p. or intravenously=i.v. injections; dose: 1-, 5-, 10-, 30-, 60-, and 120mg/kg body weight of either single metabolites or combination of all metabolites or combination of kynurenine + 3-hydroxykynurenine + 3-hydroxyanthranilic acid or combination of 3-hydroxykynurenine + 3-hydroxyanthranilic acid) from day 5-10 of pregnancy. Animals are sacrificed on day 13 and the number of normal and resorbing sites are counted (s. "Arck P. et al.", 1999, Cell. Immunol. 196:71-79). Tryptophan metabolite treatment prevents abortions in a dose-dependent manner.

### Treatment ofautoimmune diseases

EAE (Experimental Autoimmune Encephalomyelitis) in mice is induced according to the following protocol ("Menges M. et al.", 2002, J. Exp. Med. 195:15-21). On day 0, C57B1/6 mice are treated subcutaneously with 50ug MOG peptide (myelinoligodendrogliaprotein-derived peptide) (Sigma Genosys). The peptide was dissolved in 50µl Phosphate Balanced Saline (=PBS) emulsified in 50µl Complete Freund's Adjuvant that was further enriched with 10mg/ml Mycobacterium tuberculosis (H37Ra, Difco/BD Pharmingen). In addition 200ng Pertussis toxin is injected i.p. at day 0 and 2. After 7-21 days the mice develop EAE - a disease which mimics human multiple sclerosis. Paralysis is evaluated according to a score system (1-4). Controls consist of untreated mice. Tryptophan metabolites are injected daily (i.p. or i.v.; dose: 1-, 5-, 10-, 30-, 60-, and 120mg/kg of either single metabolites or combination of all metabolites or combination of kynurenine + 3-hydroxykynurenine + 3-hydroxyanthranilic acid or combination of 3-hydroxykynurenine + 3-hydroxyanthranilic acid) starting with day 0. Mice receiving tryptophan metabolites are protected from EAE in a dose-dependent manner.

### Suppression ofallograft rejection.

Tryptophan metabolites are tested in a allogeneic skin transplant model in rats (donor=BN and recipient=LEW strain). Abdominal skin of BN rats is harvested and grafts of approx. 1x1 cm are prepared and placed to the left side of thorax below the scapula of LEW rats after removing a similar piece of their own skin. The grafts are sutured, bandaged, and monitored daily until complete rejection (necrosis). Recipients are treated daily with tryptophan metabolites (i.p. or i.v. injections; dose: 1-, 5-, 10-, 30-, 60-, and 120mg/kg body weight of either single metabolites or combination of all metabolites or combination of kynurenine + 3-hydroxykynurenine + 3-hydroxyanthranilic acid or combination of 3-hydroxykynurenine + 3-hydroxyanthranilic acid) starting with day 0. Controls consist of reqipients treated with PBS or not at all. Treatment with metabolites prolong graft survival in a dose-dependent manner.

## Claims

1. The use of a compound selected from the group consisting of kynurenine, N-formylkynurenine, anthranilic acid, quinolinic acid, 3-hydroxykynurenine, and 3-hydroxyanthranilic acid in any of their stereoisomeric forms or a mixture thereof in any ratio or a pharmaceutically acceptable salt thereof for the manufacture of a medicament for immunosuppressive treatment of autoimmune diseases, rheumatic diseases, recurrent abortion, or rejection in case of cell, tissue, or organ transplantations or for the treatment of any other disease **characterized by** an increased activity of the immune system or where immunosuppression is required.

2. The use according to claim 1 wherein said compound is selected from the group consisting of kynurenine, 3-hydroxykynurenine, and 3-hydroxyanthranilic acid in any of their stereoisomeric forms or a mixture thereof in any ratio or a pharmaceutically acceptable salt thereof.

3. The use according to claim 1 or 2 wherein said compound is a mixture of 3-hydroxykynurenine and 3-hydroxyanthranilic acid in equal ratios.

4. The use of a compound according to any of claims 1 to 3 for the manufacture of a medicament for the suppression of the response of T-cells and/or B-cells and/or NK cells.

5. The use of a compound according to any of claims 1 to 3 for the manufacture of a medicament for killing T-cells and/or B-cells and/or NK cells.

6. The use of a pharmaceutical preparation comprising a pharmaceutically acceptable carrier and an effective dose of at least one compound selected from the group consisting of kynurenine, N-formylkynurenine, anthranilic acid, quinolinic acid, 3-hydroxykynurenine, and 3-hydroxyanthranilic acid in any of their stereoisomeric forms or a mixture thereof in any ratio or a pharmaceutically acceptable salt thereof for immunosuppressive treatment of autoimmune diseases, rheumatic diseases, recurrent abortion or rejection in case of cell, tissue, or organ transplantations or for the treatment of any other disease **characterized by** an increased activity of the immune system or where immunosuppression is required.

7. The use of a pharmaceutical preparation according to claim 6 for the suppression of the response of T-cells and/or B-cells and/or NK cells.

8. The use of a pharmaceutical preparation according to claim 6 for killing T-cells and/or B-cells and/or NK cells.

9. The use of a pharmaceutical preparation according to any of claims 6 to 8, which pharmaceutical preparation is in the form of a pill, tablet, lacquered tablet, sugar-coated tablet, granule, hard or soft gelatin capsule, aqueous, alcoholic or oily solution, syrup, emulsion or suspension, suppository, solution for injection or infusion, ointment, tincture, spray, transdermal therapeutic systems, nasal spray, aerosol mixture, microcapsule, implant or rod.

10. A pharmaceutical preparation comprising a pharmaceutically acceptable carrier and an effective dose of kynurenine and at least one compound selected from the group consisting of N-formylkynurenine, anthranilic acid, quinolinic acid, 3-hydroxykynurenine, and 3-hydroxyanthranilic acid wherein kynurenine, N-formylkynurenine, anthranilic acid, quinolinic acid, 3-hydroxykynurenine, and 3-hydroxyanthranilic acid can be present in any of their stereoisomeric forms or a mixture thereof in any ratio or as a pharmaceutically acceptable salt thereof.
